# EUROPEAN PATENT SPECIFICATION

(11) **EP 3 585 374 B1**
(45) Date of publication and mention of the grant of the patent: **19.07.2023**
(21) Application number: 18705170.1
(22) Date of filing: 21.02.2018
(51) Int. Cl.: A61K 31/192, A61K 31/575, A61P 1/16, A61K 9/14, A61P 29/00, A61K 45/06, A61K 9/20

(54) **COMBINATION OF A PPAR AGONIST WITH A FXR AGONIST**
KOMBINATION AUS EINEM PPAR AGONISTEN UND EINEM FXR AGONISTEN
COMBINAISON AVEC UN AGONISTE PPAR ET UN AGONISTE FXR

(30) Priority: 21.02.2017 EP 17157279; 21.03.2017 EP 17162161; 05.04.2017 EP 17165131
(43) Date of publication of application: 01.01.2020
(73) Proprietor: Genfit, 59120 Loos (FR)
(72) Inventor: NOEL, Benoît, 59147 GONDECOURT (FR); WALCZAK, Robert, 59000 Lille (FR); BELANGER, Carole, 59910 Bondues (FR)
(74) Representative: Cabinet Becker et Associés
(86) International application number: PCT/EP2018/054305
(87) International publication number: WO 2018/153933

(56) References cited:
- WO-A1-2016/154258
- WO-A2-2016/127019
- US-A1- 2006 252 670
- RATZIU VLAD: "Novel Pharmacotherapy Options for NASH", DIGESTIVE DISEASES AND SCIENCES, SPRINGER NEW YORK LLC, US, vol. 61, no. 5, 22 March 2016 (2016-03-22) , pages 1398-1405, XP035905940, ISSN: 0163-2116, DOI: 10.1007/S10620-016-4128-Z [retrieved on 2016-03-22]
- ANNA BAGHDASARYAN ET AL: "Dual farnesoid X receptor/TGR5 agonist INT-767 reduces liver injury in the Mdr2 -/- ( Abcb4 -/- ) mouse cholangiopathy model by promoting biliary HCO?3- output", HEPATOLOGY, vol. 54, no. 4, 27 September 2011 (2011-09-27), pages 1303-1312, XP055466254, US ISSN: 0270-9139, DOI: 10.1002/hep.24537

## Description

### BACKGROUND OF THE INVENTION

According to the Washington Manual of Medical Therapeutics (31st ed.; 2004; Lippincott Williams & Wilkins), liver disorders can be categorized in different groups of diseases, in particular viral diseases, drug- and alcohol-related liver diseases, immune-mediated liver diseases, metabolic liver diseases, miscellaneous diseases such as non-alcoholic fatty liver disease, and complications of hepatic insufficiency (such as fulminant hepatic failure or hepatocellular carcinoma).

In particular, non-alcoholic fatty liver disease (NAFLD) is a common hepatic disorder with histological features of alcohol-induced fatty liver disease in individuals who consume little or no alcohol (Yeh M et al., 2007; Marchesini et al., 2003). NAFLD is due to the abnormal retention of lipids within cells (commonly defined as steatosis), an event more frequent in liver since this organ is primarily responsible of lipid metabolism. NAFLD has a spectrum of histological forms including hepatic steatosis, and non-alcoholic steatohepatitis (NASH), which is characterized by liver inflammation, steatosis, necrosis and fibrosis due to the disruption of liver cells. Conditions associated with NAFLD are varied, and include type 2 diabetes, obesity, dyslipidemia, metabolic syndrome, treatment with hepatotoxic drugs, toxins, infectious agents, or other exogenous causes.

Although NAFLD typically follows a benign, non-progressive clinical course, NASH is a potentially serious condition; as many as 25% of patients may progress to advanced fibrosis, cirrhosis and experience complications of portal hypertension, liver failure and hepatocellular carcinoma, which makes an early and correct assessment mandatory (Yeh M et al, 2007).

Hepatic imaging systems are useful to evaluate also liver structure and presence of steatosis. However, liver biopsy remains the gold standard for evaluating liver fibrosis, but this method of analysis could not be done for every single study due to its invasiveness. Non-invasive evaluation of liver biochemistry and metabolism is often used to define liver diseases, such as in NAFLD and NASH (Gressner A et al., 2009; Vuppalanchi R and Chalasani N, 2009). By using plasma, high level of enzymes such as Alanine aminotransferase (ALAT), Aspartate aminotransfersase (ASAT), Alkaline Phosphatase (AP), and/ or Gamma Glutamyl Transpeptidase (GGT), as well as the presence of other proteins of liver origin (including haptoglobin, total bilirubin, alpha-2-microglobulin, Resistin, cleaved or intact cytokeratin-18) are commonly measured in addition to serum glucose and insulin resistance parameters. Since the level of ALAT activity is frequently increased in NASH patients (Angulo P et al, 2002), this criteria is considered as a surrogate marker for assessing liver injury. In fact, reliable non-invasive methods are not available to correctly diagnose NAFLD or NASH and even the histological features are not always sufficient to distinguish properly NAFLD or NASH from other conditions such as alcoholic liver disease (Yeh M et al., 2007, Vuppalanchi R and Chalasani N, 2009).

Means for an effective treatment for liver fibrotic diseases, and NAFLD and NASH in particular, are still insufficient. No treatment is established for patient with NASH, and several therapeutic options are tested in clinical trial (Vuppalanchi R and Chalasani N, 2009, Dowman J.K et al., 2009). These studies involve the use of many different families of chemical compounds (fibrates, thiazolidinediones, biguanides, statins, cannabinoids) and therapeutic targets (nuclear receptors, angiotensin receptors, cannabinoid receptors, HMG-CoA reductase). Recently, studies involving thiazolidinediones (Rosiglitazone and Pioglitazone) have shown that these drugs may improve liver condition but treatment with these drugs is not without undesired effects such as higher risks of congestive cardiac failure and osteoporosis, as well as weight gain with psychological effects on the patient (Dowman J.K et al., 2009; Shiri-Sverdlov R et al., 2006; Neuschwander-Tetri et al., 2003). Clinical trials involving the administration of cannabinoids have raised the concern of neuropsychiatric disruption (Vuppanchi R and Chalasani N, 2009). Other therapies currently ongoing are seeking to assess in NASH drugs as antioxidants but none of these treatments has yet showed convincing results (Nelson A et al., 2009).

Currently there are no approved treatments of NASH, but two compounds with different mechanisms of action are currently in Phase 3 clinical trials - obeticholic acid (OCA, FXR agonist) and Elafibranor.

WO2016154258 relates methods of treating liver disease using indane acetic acid derivatives. US2006252670 relates to methods of reducing PPARy-induced side effects in a patient, by administration of a FXR agonist. Ratziu et al. (Dig Dis Sci, 2016, 61: 1398-1405) discusses pharmacotherapy options in NASH. WO2016127019 relates to pharmaceutical compositions for combination therapy, comprising a combination of a FXR agonist and at least one lipid lowering agent. Baghdasaryan et al. (Hepatology, 2011, 54(4): 1303-1312) reports that the dual FXR/TGR5 agonist INT-767 reduces liver injury in the *Mdr2*^{*-*/*-*} (*Abcb4*^{*-*/*-*}) mouse cholangiopathy model by promoting biliary HCO₃⁻ output.

1-[4-methylthiophenyl]-3-[3,5-dimethyl-4-carboxydimethylmethyloxyphenyl]prop-2-en-1-one (Elafibranor, or ELA formely named GFT505), a PPAR-alpha/delta dual agonist disclosed in WO2004005233, possesses properties which can be advantageous for the treatment of a number of gastroenterology and liver diseases, in particular cholestatic diseases such as PBC (primary biliary cholangitic) and PSC (primary sclerosing cholangitis), or liver diseases, in particular non-alcoolic fatty liver diseases (NAFLD) such as Non-Alcoolic Steato Hepatitis (NASH).

Elafibranor has been tested for clinical efficacy in NASH in a 1-year liver biopsy-based Phase 2b trial (GFT505-2127), one of the largest interventional studies ever conducted in NASH. Administered to over 800 patients and healthy volunteers to date, elafibranor has demonstrated beneficial properties for NASH, including in particular: improvement of markers of liver dysfunction, including ALAT, ASAT, γGT, ALP; improvement of insulin sensitivity and glucose homeostasis; favorable effects on plasma lipids, including decrease of plasma triglycerides and LDL-C, and increase of HDL-C levels; antiinflammatory properties; efficacy on histological NASH parameters (steatosis, inflammation, fibrosis) in animal disease models - anti-fibrotic activities; and the absence of safety concern has been confirmed in a full toxicological package up to 2-year carcinogenicity studies. Elafibranor is currently being evaluated in a clinical phase 3 study for the treatment of NASH. Evaluation of this molecule for the treatment of PBC in a clinical phase 2 study has also started.

### SUMMARY OF THE INVENTION

The present invention relates to the subject-matter defined in the claims.

The present disclosure provides a combination product comprising:
(i) a PPAR agonist; and
(ii) a FXR agonist.

In the present invention, the PPAR agonist is the PPARα/δ agonist Elafibranor (ELA in the following description) or a pharmaceutically acceptable salt thereof.

In the present invention, the FXR agonist is Obeticholic acid (or OCA in the following description) or a pharmaceutically acceptable salt thereof, or INT-767 or a pharmaceutically acceptable salt thereof,

In a further particular embodiment, the combination product of the invention is a composition comprising:
(i) ELA or a pharmaceutically acceptable salt thereof;
(ii) a FXR agonist, wherein the FXR agonist is OCA or a pharmaceutically acceptable thereof or INT-767 or a pharmaceutically acceptable salt thereof, in particular OCA or a pharmaceutically acceptable salt thereof; and
a pharmaceutically acceptable carrier.

In another particular embodiment, the combination product is a kit of parts comprising:
(i) ELA or a pharmaceutically acceptable salt thereof; and
(ii) OCA or a pharmaceutically acceptable salt thereof.

In a further particular embodiment, the combination product of the invention is a composition comprising:
(i) ELA or a pharmaceutically acceptable salt thereof;
(ii) INT-767 or a pharmaceutically acceptable salt thereof; and
a pharmaceutically acceptable carrier.

In another particular embodiment, the combination product is a kit of parts comprising:
(i) ELA or a pharmaceutically acceptable salt thereof; and
(ii) INT-767 or a pharmaceutically acceptable salt thereof).

The kit of parts of the invention is for sequential, separate or simultaneous use in the treatment of any of the diseases mentioned herein, in particular for the treatment of NAFLD, NASH, liver fibrosis, liver cirrhosis, PBC or PSC.

It is also herein disclosed a composition for use in a method for the treatment of a number of diseases, including inflammatory, metabolic, fibrotic and cholestatic diseases, comprising administering the combination product of the invention to a subject in need thereof.

In a particular embodiment, (i) and (ii) are used in a synergistic effective amount, wherein the combined effect of the amounts of (i) and (ii) is greater than the sum of the therapeutic effects of the amounts of (i) and (ii) individually administered. Thanks to the invention, the amount of each of compounds (i) and (ii) administered to the patient may be reduced in comparison to the amount of said compounds (i) and (ii) administered individually, in at least a factor 1.5, at least a factor 2 or even at least of factor 3 or higher such as in at least a factor 4, 5, 6, 7, 8, 9 or 10. For example, the amount of ELA administered to patients in clinical trials is of 80 or 120 mg/day and the amount of OCA administered to patients in clinical trials is of 10 or 25 mg/day, and the synergistic amount according to the invention, with a factor 3 reduction may be of 27 mg or 40 mg/day of ELA and of 3 and 8 mg/day for OCA.

In a particular embodiment, the patient being treated with the combination product of the invention is a patient having NAFLD, NASH, liver fibrosis, liver cirrhosis, PBC or PSC.

In another aspect, the invention relates to the combination product of the invention, for use in a method for the treatment of a disease, such as an inflammatory, metabolic, fibrotic or cholestatic disease. In this aspect, each of the components of the combination product may be used in a synergistic effective amount. In a further embodiment, the disease is NAFLD, NASH, liver fibrosis, liver cirrhosis, PBC or PSC.

In a further aspect, the invention relates to the use of the combination product of the invention in the manufacture of a medicament for the treatment of a disease, such as an inflammatory, metabolic, fibrotic or cholestatic disease. In this aspect, each of the components of the combination product may be used in a synergistic effective amount. In a further embodiment, the disease is NAFLD, NASH, liver fibrosis, liver cirrhosis, PBC or PSC.

### LEGENDS OF THE FIGURES

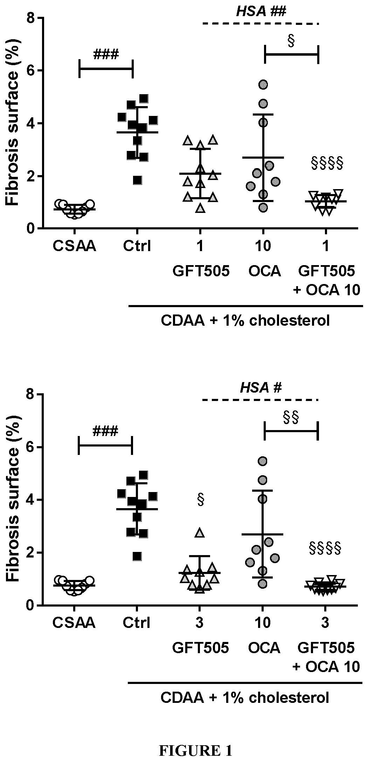

**Figure 1****:** Effect of Elafibranor (GFT505), OCA and their combination on hepatic fibrosis in CDAA/chol fed rats (n=10/group).
   Percentage of fibrosis surface was assessed by morphometric quantification of picrosirius positive area relative to the liver section area. Data are expressed as mean ± SD. # p<0.05, ## p<0.01, ### p<0.001 using Student t-test. § p<0.05, §§ p<0.01, §§§ p<0.001, §§§§ p<0.0001 using Kruskal-Wallis and uncorrected Dunn's post-hoc test. HSA, highest single agent model.

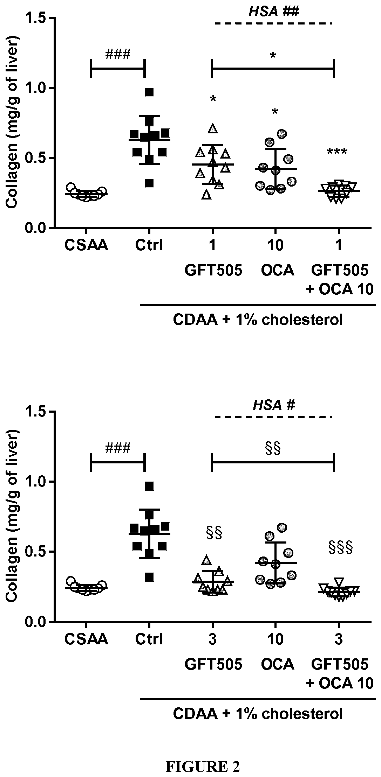

**Figure 2****:** Effect of Elafibranor (GFT505), OCA and their combination on hepatic collagen content in CDAA/chol fed rats (n=10/group).
   Data are expressed as mean ± SD. # p<0.05, ## p<0.01, ### p<0.001 using Student t-test. * p<0.05, ** p<0.01, *** p<0.001 using one-way ANOVA and uncorrected Fisher's post-hoc test. § p<0.05, §§ p<0.01, §§§ p<0.001, §§§§ p<0.0001 using Kruskal-Wallis and uncorrected Dunn's post-hoc test. HSA, highest single agent model.

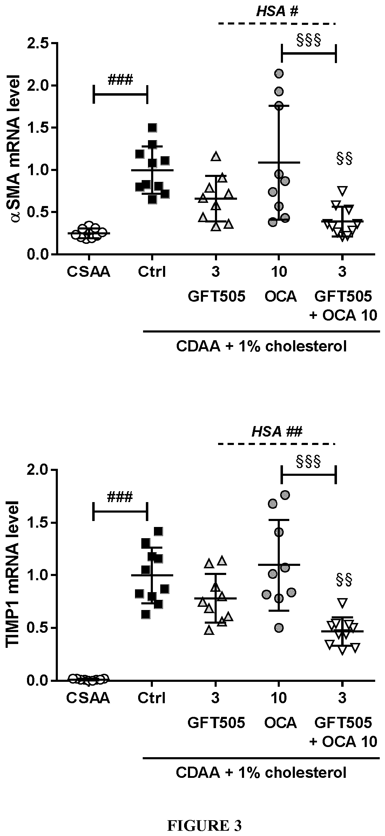

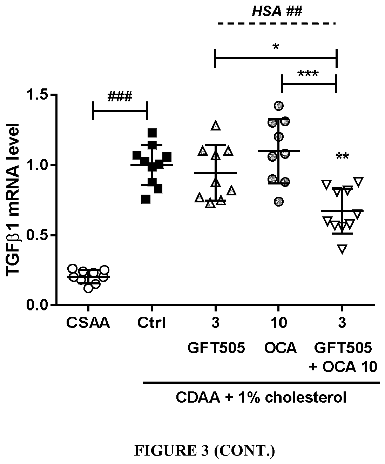

**Figure 3****:** Effect of Elafibranor (GFT505), OCA and their combination on expression of genes involved in tissue remodeling and inflammation in CDAA/chol fed rat livers (n=10/group).
   Expression of αSMA (ACTA2), TIMP1 and TGFβ was assessed by reat time squantitative PCR. Data are expressed as mean ± SD. # p<0.05, ## p<0.01, ### p<0.001 using Student t-test. * p<0.05, ** p<0.01, *** p<0.001 using one-way ANOVA and uncorrected Fisher's post-hoc test. § p<0.05, §§ p<0.01, §§§ p<0.001, §§§§ p<0.0001 using Kruskal-Wallis and uncorrected Dunn's post-hoc test. HSA, highest single agent model.

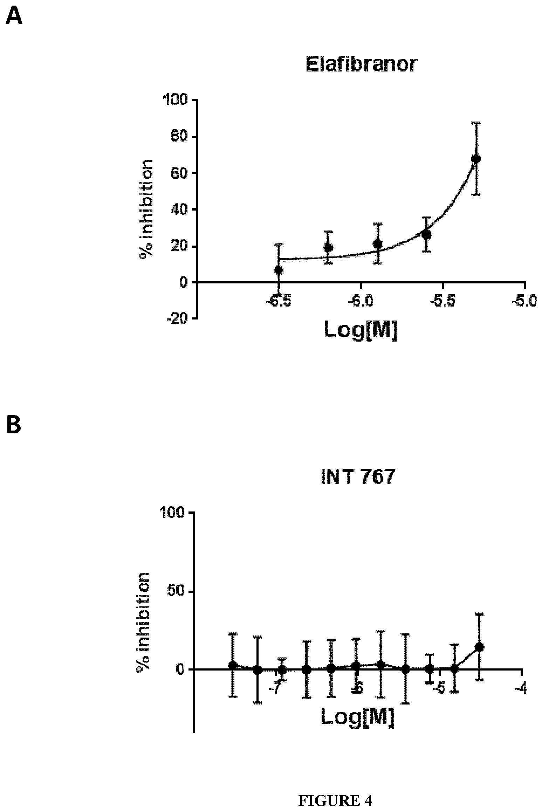

**Figure 4****:** Differential antifibrotic effect of Elafibranor and INT-767 in TGFβ-induced hHSC
   Serum-deprived HSC were preincubated for 1 hour with Elafibranor (A) and INT-767 (B) before the activation with the profibrogenic cytokine TGFβ1 (1 ng/ml). After 48 hours of incubation, the expression of α-SMA was measured by ELISA. The obtained values were transformed into percentage inhibition over TGFβ1 control. Data are presented as mean (quadruplicates) ± standard deviation (SD).

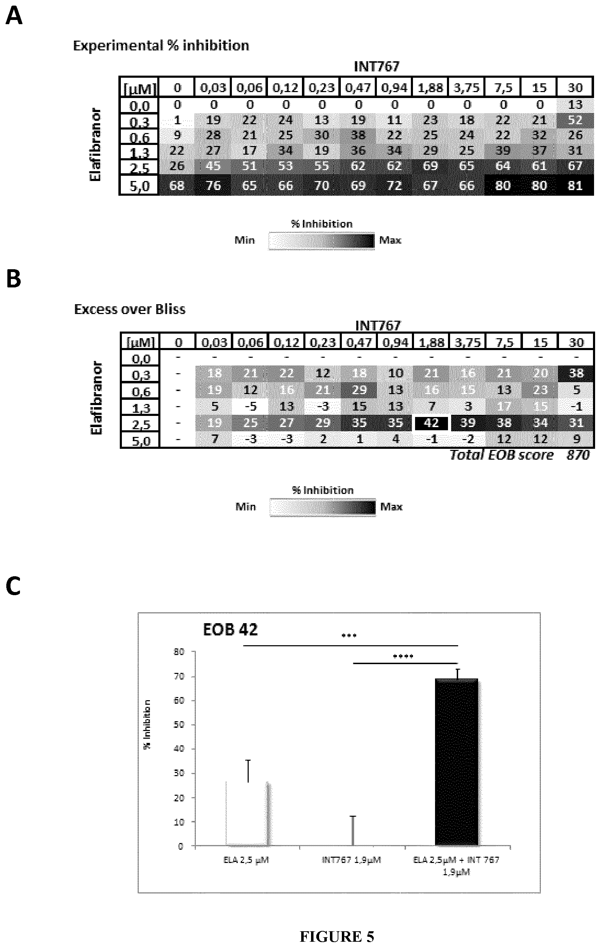

**Figure 5****:** Combination of Elafibranor with INT-767 synergistically inhibits α-SMA production in TGFβ1-induced hHSC
   Combinations were tested in a dose-response matrix format and analyzed according to the Excess Over Bliss (EOB) additivism model. Dilution series of Elafibranor (row) and INT-767 (column) were prepared, including their respective DMSO controls. The resulting mixes were added to serum-deprived HSC, 1 hour prior to the activation with the profibrogenic cytokine TGFβ1 (1 ng/ml). (A) Percentage of α-SMA inhibition over the TGFβ1 control for all combination pairs. Data are presented as mean of quadruplicates. (B) EOB scores were calculated as described in Materials and Methods. Any compound pair with EOB values > 10 was considered synergistic (colored from light grey to black). The total EOB score including all combinations was also calculated. (C) Data values derived from a synergistic combination pair were plotted in a bar graph representation. Data are presented as mean (quadruplicates) ± standard deviation (SD). * p<0.05; ** p<0.01; *** p<0.001 **** p<0.0001 using a Student's t-test to compare the combination group over the highest single agent.

### DETAILED DESCRIPTION OF THE INVENTION

As use above the term "administering" includes any mode of administration, such as oral, subcutaneous, sublingual, transmucosal, parenteral, intravenous, intra-arterial, buccal, sublingual, topical, vaginal, rectal, ophthalmic, otic, nasal, inhaled, intramuscular, intraosseous, intrathecal, and transdermal, or a combination thereof. In a preferred embodiments, the compounds are administered via oral, in particular in the form of one or more tables.

"Administering" can also include prescribing or filling a prescription for a dosage form comprising a particular compound. "Administering" can also include providing directions to carry out a method involving a particular compound or a dosage form comprising the compound.

As used herein, the term "disease" refers to a disease, disorder, condition, symptom, or indication. This term is used interchangeably with the phrase "disease or disorder".

As employed above and throughout the disclosure the term "therapeutically effective amount" refers to an amount effective, at dosages, and for periods of time necessary, to achieve the desired result with respect to the treatment of the relevant disorder, condition, or side effect. It will be appreciated that the effective amount of components of the present invention may vary from patient to patient not only with the particular compound, component or composition selected, the route of administration, and the ability of the components to elicit a desired response in the individual, but also with factors such as the disease state or severity of the condition to be alleviated, hormone levels, age, sex, weight of the individual, the state of being of the patient, and the severity of the condition being treated, concurrent medication or special diets then being followed by the particular patient, and other factors which those skilled in the art will recognize, with the appropriate dosage ultimately being at the discretion of the attendant physician. Dosage regimens may be adjusted to provide the improved therapeutic response. An effective amount is also one in which any toxic or detrimental effects of the components are outweighed by the therapeutically beneficial effects.

The term "synergistic" as used herein means that the effect achieved with the combination product and methods of this invention is greater than the sum of the effects that result from components of the combination or from methods comprising one of the components separately and in the amounts employed in the methods and compositions hereof. Such synergy may be determined according to methods well-known in the art, such as by using the Excess Over Bliss (EOB) method.

The combination product of the invention may achieve better effects than the effect achievable with each of its components used separately, in particular when used in a synergistic effective amount. A "synergistic effective amount" is an amount of each of the components of the combination product that allows achieving a synergistic effect when administered to a subject in need thereof. In other words, when components (i) and (ii) are used in a synergistic effective amount, the combined effect of the amounts of (i) and (ii) is greater than the sum of the therapeutic effects of the amounts of (i) and (ii) individually administered. As mentioned above, the amount of each of compounds (i) and (ii) administered to the patient may be reduced in comparison to the amount of said compounds (i) and (ii) administered individually. For example, the amount of each component may be reduced in at least a factor 1.5, at least a factor 2 or even at least of factor 3 or higher such as in at least a factor 4, 5, 6, 7, 8, 9 or 10. For example, the amount of ELA administered to patients in clinical trials is of 80 or 120 mg/day and the amount of OCA administered to patients in clinical trials is of 10 or 25 mg/day, and the synergistic amount according to the invention, with a factor 3 reduction may be of 27 mg or 40 mg/day of ELA and of 3 and 8 mg/day for OCA.

According to the present disclosure, the PPAR agonist is a PPAR-alpha agonist, a PPAR-gamma agonist, a PPAR-delta agonist, a PPAR-alpha/gamma dual agonist, a PPAR alpha/delta dual agonist, a PPAR gamma/delta dual agonist or PPAR alpha/gamma/delta pan agonist.

Component (ii) of the combination product is at least one PPAR-alpha/delta dual agonist

According to the present disclosure, the term "PPAR(s) agonists" refers the Peroxisome Proliferator Activated Receptor agonists, which are a class of drugs which plays a central role in lipid and glucose homeostasis. PPARα mainly influences fatty acid metabolism and its activation lowers lipid levels, while PPARγ is mostly involved in the regulation of the adipogenesis, energy balance, and lipid biosynthesis. PPARδ participates in fatty acid oxidation, mostly in skeletal and cardiac muscles, but it also regulates blood glucose and cholesterol levels.

According to the present disclosure, the term "PPAR alpha agonist" as used herein includes, but is not limited to fenofibrate, ciprofibrate, pemafibrate, gemfibrozil, clofibrate, binifibrate, clinofibrate, clofibric acid, nicofibrate, pirifibrate, plafibride, ronifibrate, theofibrate, tocofibrate, and SR10171.

According to the present disclosure, the term "PPAR gamma agonist" as used herein includes, but is not limited to Rosiglitazone, Pioglitazone, deuterated pioglitazone, efatutazone, ATx08-001, OMS-405, CHS-131, THR-0921, SER-150-DN, KDT-501, GED-0507-34-Levo, CLC-3001, and ALL-4.

According to the present disclosure, the term "PPAR delta agonist" as used herein includes, but is not limited to GW501516 (Endurabol or ({4-[({4-methyl-2-[4-(trifluoromethyl)phenyl]-1,3-thiazol-5-yl}methyl)sulfanyl]-2-methylphenoxy}acetic acid)) or MBX8025 (Seladelpar or {2-methyl-4-[5-methyl-2-(4-trifluoromethyl- phenyl)-2H-[1,2,3]triazol-4-ylmethylsylfanyl]-phenoxy}-acetic acid) or GW0742 ([4-[[[2-[3-fluoro-4- (trifluoromethyl)phenyl]-4-methyl-5-thiazolyl]methyl]thio]-2-methyl phenoxy]acetic acid) or L165041 or HPP-593 orNCP-1046.

According to the present disclosure, the term "PPAR alpha/gamma agonist" (also named glitazars) used herein includes, but is not limited to Saroglitazar, Aleglitazar, Muraglitazar, Tesaglitazar, DSP-8658.

According to the present disclosure, the term "PPAR alpha/delta agonist" used herein includes, but is not limited to ELA or T913659.

According to the present disclosure, the term "PPAR gamma/delta agonist" used herein includes, but is not limited to a conjugated linoleic acid (CLA), T3D-959.

According to the present disclosure, the term "PPAR alpha/gamma/delta agonist" used herein includes, but is not limited to IVA337 (Lanifibranor) or TTA (tetradecylthioacetic acid) or Bavachinin or GW4148 or GW9135, or Bezafibrate or Lobeglitazone, or CS038, or 2-(4-(5,6-methylenedioxybenzo[d]thiazol-2-yl)-2-methylphenoxy)-2-methylpropanoic acid (MHY2013).

The PPAR agonist may be in the form of a salt, hydrate, solvate, polymorph, or a co-crystal. The PPAR agonist may also be in the form of a hydrate, solvate, polymorph, or a co-crystal of a salt.

According to the invention, the PPAR agonist is ELA, or a pharmaceutically acceptable salt thereof. ELA has the following structure:

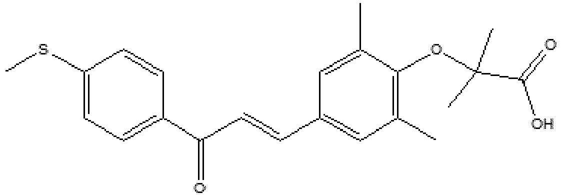

ELA may be prepared by methods described in WO2004/005233, WO2005/005369 or WO2011/144579.

A FXR agonist may be a steroidal or non-steroidal FXR agonist.

Illustrative FXR agonists are disclosed in WO02072598, WO2005082925, WO03080803, WO04007521, WO04046162, WO04045511, WO04048349, WO05082925, WO07140174, WO08000643, WO08025540, WO08025539, WO07140183, WO08157270, WO09005998, WO09027264, WO09062874, WO09080555, WO09149795, WO10034649, WO10034657, WO11020615, WO11039130, WO12087519, WO13007387, WO14184271, WO15138986, WO16073767, WO16086115, WO16086134, WO16086169 and WO16086218. According to the present disclosure, each embodiment and each specific FXR agonist disclosed in these references are individually disclosed herein in combination with a PPAR agonist.

According to a particular example, the FXR agonist is selected in the group consisting of obeticholic acid (INT-747), GS-9674, LJN-452 or LJN452, LJN-763, LMB763, EDP-305,AKN-083, INT-767, GNF-5120, LY2562175, INV-33, NTX-023-1, EP-024297, EPY-001, Px-103 and SR-45023. In a further particular example, the PPAR agonist is ELA and the FXR agonist is selected in the group consisting of obeticholic acid (INT-747), GS-9674, LJN-452 or LJN452, LJN-763, LMB-763, EDP-305, AKN-083, INT-767, GNF-5120, LY-2562175, INV-33, NTX-023-1, EP-024297, EPY-001, Px-103 and SR-45023.

In a particular embodiment, the FXR agonist is INT-767 having the following structure:

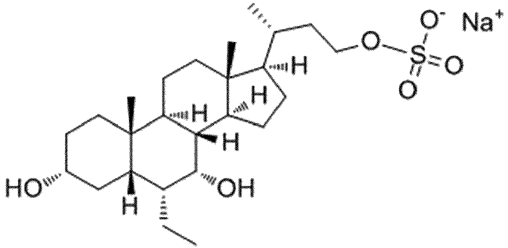

In a particular embodiment, the FXR agonist is obeticholic acid (OCA ; 6α-ethyl chenodeoxycholic acid ; INT-747) or a pharmaceutically acceptable salt thereof. OCA has the following chemical structure:

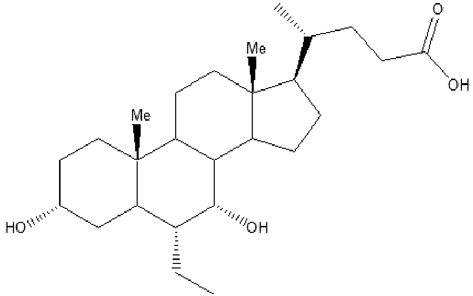

OCA may be prepared by methods described in WO2006122977.

The combination product of the invention may be used for the inhibition of proliferation and/or activation of fibroblasts responsible for the production of collagen fibers and/or responsible for the production of the extracellular matrix.

The combination product of the invention may be used for the treatment of a disease, such as a immune (e.g. autoimmune), inflammatory, metabolic, fibrotic or cholestatic disease.

According to the present invention, the term "autoimmune diseases" is used to designate a condition that arises from an abnormal immune response of the body against substances and tissues normally present in the body. The disease may be restricted to certain organs (e.g in type I diabetes or autoimmune thyroiditis) or involve a particular tissue in different places (e.g. in Goodpasture's disease, affection of the basement membrane in the lung and the kidney).

The term "inflammation" is used to designate a condition that arise from a protective response involving host cells, blood vessels, and proteins and other mediators which may serve to eliminate the cause of cell/tissue injury, as well as the necrotic cells/tissues resulting from the original insult, and to initiate the process of repair. The inflammatory reaction may be manifested by pain, heat, redness, swelling, blood vessels dilatation, blood flow increase and loss of function.

The terms "fibrosis", "fibrotic disease", "fibrotic disorder" and declinations thereof denote a pathological condition of excessive deposition of fibrous connective tissue in an organ or tissue. More specifically, fibrosis is a pathologic process, which includes a persistent fibrotic scar formation and overproduction of extracellular matrix, by the connective tissue, as a response to tissue damage. Physiologically, the deposit of connective tissue can obliterate the architecture and function of the underlying organ or tissue.

According to the present invention, the fibrosis may be any organ or tissue fibrosis. Illustrative, non-limiting examples of particular organ fibrosis include liver, kidney, skin, epidermis, endodermis, muscle, tendon, cartilage, heart, pancreas, lung, uterus, nervous system, testis, ovary, adrenal gland, artery, vein, colon, intestine (e.g. small intestine), biliary tract, soft tissue (e.g. mediastinum or retroperitoneum), bone marrow, joint or stomach fibrosis.

In a preferred embodiment, the fibrotic disorder is selected in the group consisting of a liver, gut, lung, heart, kidney, muscle, skin, soft tissue (e.g. mediastinum or retroperitoneum), bone marrow, intestinal, and joint (e.g. knee, shoulder or other joints) fibrosis.

In a more preferred embodiment, the fibrotic disorder is selected in the group consisting of the liver, lung, skin, kidney and intestinal fibrosis.

In a more preferred embodiment of the present invention, treated fibrotic disorder is selected in the group consisting of the following non exhaustive list of fibrotic disorders: non-alcoholic steatohepatitis (NASH), pulmonary fibrosis, idiopathic pulmonary fibrosis, skin fibrosis, eye fibrosis, endomyocardial fibrosis, mediastinal fibrosis, myelofibrosis, retroperitoneal fibrosis, progressive massive fibrosis (a complication of coal workers' pneumoconiosis), proliferative fibrosis, neoplastic fibrosis, lung fibrosis consecutive to chronic inflammatory airway disease (COPD, asthma, emphysema, smoker's lung,tuberculosis, IPF), alcohol or drug-induced liver fibrosis, liver cirrhosis, infection-induced liver fibrosis, radiation or chemotherapeutic-induced fibrosis, nephrogenic systemic fibrosis, Crohn's disease, ulcerative colitis, keloid, old myocardial infarction, scleroderma/systemic sclerosis, arthrofibrosis, some forms of adhesive capsulitis, chronic fibrosing cholangiopathies such as Primary Sclerosing Cholangitis (PSC) and Primary Biliary Cholangitis (PBC), biliary atresia, familial intrahepatic cholestasis type 3 (PFIC3), peri-implantational fibrosis and asbestosis.

Cholestasis is defined as a decrease in bile flow due to impaired secretion by hepatocytes (hepatocellular cholestasis) or to obstruction of bile flow through intra-or extrahepatic bile ducts (obstructive cholestasis). In clinical practice, cholestasis is any condition in which the flow of bile from the liver is slowed or blocked.

Examples of inflammatory diseases, fibrotic diseases, metabolic diseases and cholestatic diseases include metabolic liver diseases, non-alcoholic fatty liver disease (NAFLD), non-alcoholic steatohepatitis (NASH), drug-induced liver diseases, alcohol-induced liver diseases, infectious agent induced liver diseases, inflammatory liver diseases, immune system dysfunction-mediated liver diseases, dyslipidemia, cardiovascular diseases, restenosis, syndrome X, metabolic syndrome, diabetes, obesity, hypertension, chronic cholangiopathies such as Primary Sclerosing Cholangitis (PSC), Primary Biliary Cholangitis (PBC), biliary atresia, familial intrahepatic cholestasis type 3 (PFIC3), inflammatory bowel diseases, Crohn's disease, ulcerative colitis, keloid, old myocardial infarction, scleroderma/systemic sclerosis, inflammatory diseases, neurodegenerative diseases, cancers, liver cancer, hepatocallular carcinoma, gastrointestinal cancer, gastric cancer, meningioma associated with neurofibromatosis, pancreatic neuroendocrine tumors, pancreatic exocrine tumors, leukemia, myeloproliferative/myelodisplastic diseases, mastocytosis, dermatofibrosarcoma, solid tumors including breast, lung, thyroid or colorectal cancer, a prostate cancer, liver fibrosis or cirrhosis of any origin, metabolic disease-induced liver fibrosis or cirrhosis, NAFLD-induced fibrosis or cirrhosis, NASH-induced fibrosis or cirrhosis, alcohol-induced liver fibrosis or cirrhosis, drug-induced liver fibrosis or cirrhosis, infectious agent-induced liver fibrosis or cirrhosis, parasite infection-induced liver fibrosis or cirrhosis, bacterial infection-induced liver fibrosis or cirrhosis, viral infection-induced fibrosis or cirrhosis, HBV-infection induced liver fibrosis or cirrhosis, HCV-infection induced liver fibrosis or cirrhosis, HIV-infection induced liver fibrosis or cirrhosis, dual HCV and HIV-infection induced liver fibrosis or cirrhosis, radiation- or chemotherapy-induced fibrosis or cirrhosis, biliary tract fibrosis, liver fibrosis or cirrhosis due to any chronic cholestatic disease, gut fibrosis of any etiology, Crohn's disease-induced fibrosis, ulcerative colitis-induced fibrosis, intestine (e.g. small intestine) fibrosis, colon fibrosis, stomach fibrosis, skin fibrosis, epidermis fibrosis, endodermis fibrosis, skin fibrosis due to scleroderma/systemic sclerosis, lung fibrosis, lung fibrosis consecutive to chronic inflammatory airway diseases, such as COPD, asthma, emphysema, smoker's lung, tuberculosis, pulmonary fibrosis, idiopathic pulmonary fibrosis (IPF), heart fibrosis, kidney fibrosis, nephrogenic systemic fibrosis, muscle fibrosis, soft tissue (e.g. mediastinum or retroperitoneum) fibrosis, bone marrow fibrosis, joint fibrosis, tendon fibrosis, cartilage fibrosis, pancreas fibrosis, uterus fibrosis, nervous system fibrosis, testis fibrosis, ovary fibrosis, adrenal gland fibrosis, artery fibrosis, vein fibrosis, eye fibrosis, endomyocardial fibrosis, mediastinal fibrosis, myelofibrosis, retroperitoneal fibrosis, progressive massive fibrosis (a complication of coal workers' pneumoconiosis), proliferative fibrosis, neoplastic fibrosis, peri-implantational fibrosis and asbestosis, arthrofibrosis, adhesive capsulitis.

In a particular embodiment, the disease is selected in the group consisting of metabolic liver diseases, non-alcoholic fatty liver disease (NAFLD), non-alcoholic steatohepatitis (NASH), drug-induced liver diseases, alcohol-induced liver diseases, infectious agent induced liver diseases, inflammatory liver diseases, immune system dysfunction-mediated liver diseases, dyslipidemia, cardiovascular diseases, restenosis, syndrome X, metabolic syndrome, diabetes, obesity, hypertension, chronic cholangiopathies such as Primary Sclerosing Cholangitis (PSC), Primary Biliary Cholangitis (PBC), biliary atresia, familial intrahepatic cholestasis type 3 (PFIC3), inflammatory bowel diseases, Crohn's disease, ulcerative colitis, liver cancer, hepatocallular carcinoma, gastrointestinal cancer, gastric cancer, colorectal cancer, metabolic disease-induced liver fibrosis or cirrhosis, NAFLD-induced fibrosis or cirrhosis, NASH-induced fibrosis or cirrhosis, alcohol-induced liver fibrosis or cirrhosis, drug-induced liver fibrosis or cirrhosis, infectious agent-induced liver fibrosis or cirrhosis, parasite infection-induced liver fibrosis or cirrhosis, bacterial infection-induced liver fibrosis or cirrhosis, viral infection-induced fibrosis or cirrhosis, HBV-infection induced liver fibrosis or cirrhosis, HCV-infection induced liver fibrosis or cirrhosis, HIV-infection induced liver fibrosis or cirrhosis, dual HCV and HIV-infection induced liver fibrosis or cirrhosis, radiation- or chemotherapy-induced fibrosis or cirrhosis, biliary tract fibrosis, liver fibrosis or cirrhosis due to any chronic cholestatic disease, gut fibrosis of any etiology, Crohn's disease-induced fibrosis, ulcerative colitis-induced fibrosis, intestine (e.g. small intestine) fibrosis, colon fibrosis, stomach fibrosis, lung fibrosis, lung fibrosis consecutive to chronic inflammatory airway diseases, such as COPD, asthma, emphysema, smoker's lung, tuberculosis, pulmonary fibrosis, idiopathic pulmonary fibrosis (IPF).

In a further embodiment, the disease is NAFLD, NASH, liver fibrosis, liver cirrhosis, PBC or PSC

The term "treatment" or "treating" refers to therapy, prevention, or prophylaxis of a disorder in a subject in need thereof. The treatment involves the administration of a the combination product of the invention to subjects (e.g. patients) having a declared disorder to prevent, cure, delay, reverse, or slow down the progression of the disorder, improving thereby the condition of patients. A treatment may also be administered to subjects that are either healthy or at risk of developing a disorder such as an immune (e.g. autoimmune), inflammatory, fibrotic or cholestatic disorder.

The term "subject" refers to a mammal and more particularly a human. The subjects to be treated according to the invention can be appropriately selected on the basis of several criteria associated with immune (e.g. autoimmune), inflammatory, fibrotic and cholestatic pathological processes such as previous and/or present drug treatments, associated pathologies, genotype, exposure to risk factors, as well as any other relevant biomarker that can be evaluated by means of any suitable immunological, biochemical, or enzymatic method.

The inventors herein show that the combination of a PPAR agonist and of a FXR agonist, in particular the combination of ELA and OCA or INT-767, in particular the combination of ELA and OCA, would benefit a wider patient population and can have a synergistic effect, thereby allowing therapeutic dose reduction. The associated therapeutic dose reduction may decrease the incidence of adverse drug effects. Accordingly, the invention also relates to the a method for the treatment of a disease as defined above, with a decreased incidence of adverse drug effects.

The frequency and/or amount relative to the administration can be adapted by one of ordinary skill in the art, in function of the patient, the pathology, the form of administration, etc. Typically, the combination product of the present invention can be administered for the treatment of a disease at a dose for ELA comprised between 10 mg/day to 1000 mg/day, such as from 50 mg/day to 500 mg/day, and particularly from 70 mg/day to 150 mg/day. The dose of OCA may be comprised between 5mg/day to about 100 mg/day, such as a dose from about 10 mg/day to about 50 mg/day. In a particular embodiment of the invention, OCA is used in combination with ELA at a dose comprised between 10 mg/day to 25 mg/day for OCA and 80 to 120 mg/day for ELA. In a particular embodiment, OCA is used at 10 or 25 mg/day and ELA is used at 80 or 120 mg/day.

In another embodiment, the amount of both the PPAR agonist and the FXR agonist is a synergistic effective amount. Particular embodiments include a reduction of a factor 1.5, 2, 2.5, 3, 3.5, 4, 4.5, 5, 5.5, 6, 6.5, 7, 7.5, 8, 8.5, 9, 8.5, 10 or of more than 10 of the PPAR agonist and/or of the FXR agonist. In a further particular embodiment implementing ELA (or a pharmaceutically effective amount thereof) as the PPAR agonist and OCA or a pharmaceutically acceptable salt thereof, INT-767 or a pharmaceutically acceptable salt thereof, or LJN452 or a pharmaceutically acceptable salt thereof, as the FXR agonist includes a reduction of a factor 1.5, 2, 2.5, 3, 3.5, 4, 4.5, 5, 5.5, 6, 6.5, 7, 7.5, 8, 8.5, 9, 8.5, 10 or of more than 10 of ELA and/or of the FXR agonist. Further particular embodiments implementing ELA (or a pharmaceutically acceptable salt thereof) as the PPAR agonist and OCA (or a pharmaceutically acceptable salt thereof) as the FXR agonist includes:
- a reduction of a factor 1.5 of the amount of both components, with ELA used at a dose comprised between 5 and 80 mg/day and OCA used at a dose comprised between 7 and 17 mg/day;
- a reduction of a factor 2 of the amount of both components, with ELA used at a dose comprised between 40 and 60 mg/day and OCA used at a dose comprised between 5 and 12.5 mg/day;
- a reduction of a factor 2.5 of the amount of both components, with ELA used at a dose comprised between 32 and 48 mg/day and OCA used at a dose comprised between 4 and 10 mg/day;
- a reduction of a factor 3 of the amount of the amount of both components, with ELA used at a dose comprised between 27 and 40 mg/day and OCA used at a dose comprised between 3 and 8 mg/day;
- a reduction of a factor 5 of the amount of the amount of both components, with ELA used at a dose comprised between 16 and 24 mg/day and OCA used at a dose comprised between 2 and 5 mg/day;
- a reduction of a factor 10 of the amount of the amount of both components, with ELA used at a dose comprised between 8 and 12 mg/day and OCA used at a dose comprised between 1 and 2.5 mg/day;
- etc.

In a particular embodiment, the amount of each of the components of the combination product of the invention is administered as a single dosage, once a day, or by administering several times the components, for example by administering half of the daily amount twice a day, such as during meals (for example during lunch and dinner).

In a particular embodiment, the PPAR agonist and the FXR agonist are administered simultaneously, sequentially or separately. In a particular embodiment, the PPAR agonist and the FXR agonist are administered sequentially, the PPAR agonist being administered first, and then the FXR agonist, or the FXR agonist being administered first and then the PPAR agonist.

The time of treatment may vary to a large extent depending on the condition to be treated and the stage of said condition. For example, the combination product may be administered at least two or more consecutives days, such as at least 7, 8, 9 or 10 days or more; at least one week or more, such as at least 1, 2, 3, 4, 5, 10, 20, 50, 60, 70 or 72 weeks or more; at least one month or more, such as at least 1, 2, 3, 4, 5, 10, 15, 20 or 24 months or more; or at least one year or more, such as at least 1, 2, 3, 4 or 5 years or more.

The invention is further described with reference to the following, non-limiting, examples.

### EXAMPLES

### Materials and methods

### Evaluation of Elafibranor, OCA and the combination Elafibranor + OCA in a chronic CDAA + 1% cholesterol model (12 weeks)

The preventive effects of Elafibranor (GFT505) alone, OCA alone and the combination of both were assessed in a fibrosing NASH -model of rats fed a CDAA + 1% cholesterol diet. 150-175g male Wistar rats were fed a control (CSAA) diet, CDAA + 1% cholesterol diet, or CDAA + 1% cholesterol diet supplemented with Elafibranor 1, 3 and 10mg/kg/day, OCA 10 and 30mg/kg/day or combined drugs (Elafibranor 1, 3 and 10mg/kg/day combined to OCA 10mg/kg/day) for 12 weeks.

The body weight and the food intake were monitored twice per week. On the last day of treatment, rats were sacrificed after a 6h fasting period. The liver was rapidly excised for biochemical and histological studies.

All animal procedures were performed according to standard protocols and in accordance with the standard recommendations for the proper care and use of laboratory animals.

### Histology

### Tissue embedding and sectioning:

The liver slices were first fixed for 12 hours in formalin 4% solution. Then, the liver pieces were washed 30 minutes in PBS, and dehydrated in ethanol solutions (successive baths at 70, 80, 95 and 100% ethanol). The liver pieces were incubated in three different baths of Xylene (Sigma-Aldrich cat# 534056), followed by two baths in liquid paraffin (56°C). Liver pieces were then put into racks that were gently filled with Histowax^{®} to completely cover the tissue.

The paraffin blocks containing the tissue pieces were removed from the racks and stored at room temperature. The liver blocks were cut into 3 µm slices.

### Hematoxylin /Eosin staining

Liver sections were deparaffinized, rehydrated and incubated for 3 minutes in Mayer's Hematoxylin (Microm, cat #F/C0303). Then, the liver sections were rinsed in water and incubated 1 minute in Eosin G (VWR, cat# 1.09844.1000). Sections were rinsed in water then dehydrated, and mounted using the CV Mount medium (Leica, cat #14046430011).

### Picrosirius red staining

Liver sections were deparaffinized, rehydrated and incubated for 15 minutes in a solution of Fast Green FCF 0.1% (Sigma-Aldrich, cat# F7258) before rinsing in a bath of 0.5% acetic acid (Panreac, cat# 131008.1611). Then, the liver sections were rinsed in water and incubated 30 minutes in a solution of 0.1% sirius red (Direct Red 80, Fluka cat# 43665) in saturated aqueous picric acid (Sigma-Aldrich cat# P6744). Sections were then dehydrated, and mounted using the CV Mount medium (Leica, cat #14046430011).

### Histological examinations

A technician blinded to the source of each liver specimen performed histological examinations. Virtual slides were generated using the Pannoramic 250 scanner from 3D Histech. For each animal, a score summarizing the main histological lesions of NASH was attributed according to the NASH Clinical Research Network (Kleiner 2005, Brunt 1999). Briefly, steatosis, lobular inflammation and hepatocytes ballooning were scored. The NAFLD Activity Score (NAS score) was established for each individual as the unweighted sum of the steatosis, lobular inflammation and the ballooning injury grading.

Using Quant Center software (3D Histech, including Pattern Quant and Histo Quant modules), collagen-stained areas were quantified. Briefly, Pattern Quant was used to detect the tissue and measure its surface. Then, Histo Quant was used to detect the stained collagen content and measure its surface, based on a color threshold method. The fibrosis area was then expressed as the percentage of the collagen surface to the whole tissue per animal.

### Measurement of hepatic collagen content

The hepatic collagen content was determined using the appropriate QuickZyme kit (Total collagen assay, cat# QZB-totcol2). The assay is based on the detection of hydroxyproline, which is a non-proteinogenic amino acid mainly found in the triple helix of collagen. Thus, hydroxyproline in tissue hydrolysates can be used as a direct measure of the amount of collagen present in the tissue (without discrimination between procollagen, mature collagen and collagen degradation products).

Complete hydrolysis of tissue samples in 6M HCl at 95°C is required before dosing the hydroxyproline. The assay results in the generation of a chromogen with a maximum absorbance at 570 nm. Results are expressed as mg of collagen / g of liver.

### Hepatic gene expression analysis

Total RNA was isolated from rat livers using RNeasy Mini Kit (Qiagen) following manufacturer's instructions. Total RNA were reverse transcribed into cDNA using M-MLV RT (Moloney Murine Leukemia Virus Reverse Transcriptase) (Invitrogen cat# 28025) in 1x RT buffer (Invitrogen), 0.5mM DTT (Invitrogen), 0.18mM dNTPs (Promega), 200ng pdN6 (Amersham) and 30U of RNase inhibitor (Promega).

Quantitative PCR was then carried out using the CFX96 Touch^{™} Real-Time PCR Detection System (Biorad). Briefly, the PCR reactions were performed in 96-WP format in 25µl of total volume containing 1µL of reverse transcription reaction, 0.5µL of reverse and forward primers (10 pmol each), and 12,5µl of 2X iQ SYBR Green Supermix (BioRad), using the following primer sequences:

| **Gene** | **Forward** | **Reverse** |
|---|---|---|
| RPLP0 | CATGCTCAACATCTCCCCCTTCTCC (SEQ ID NO:1) | GGGAAGGTGTAATCCGTCTCCACAG (SEQ ID NO:2) |
| αSMA (ACTA2) | ACTGGGACGACATGGAAAAG (SEQ ID NO:3) | CATCTCCAGAGTCCAGCACA (SEQ ID NO:4) |
| TIMP1 | TCCCCAGAAATCATCGAGAC (SEQ ID NO:5) | TCAGATTATGCCAGGGAACC (SEQ ID NO:6) |
| TGFB1 | TGAGTGGCTGTCTTTTGACG (SEQ ID NO:7) | TGGGACTGATCCCATTGATT (SEQ ID NO:8) |
| CCR5 | CAGAACAGTCAACTTTGGGG (SEQ ID NO:9) | ACGTGGAAAATGAGGACTGC (SEQ ID NO:10) |

Expression levels were normalized using the expression of Rplp0 gene as a reference in samples.

For each gene, the standard curves were drawn by selecting the best points (at least three points) in order to have PCR reaction efficiency close to 100% and a correlation coefficient close to 1. Expression levels were determined using the standard curve equation for both the housekeeping gene and the target gene (taking into account the specific PCR efficiency of each target gene).

### Evaluation of the combination of Elafibranor and another FXR agonist in a vitro model of hepatic fibrogenesis

### hHSC culture

The human primary hepatic stellate cells (hHSC) (Innoprot) were cultured in STeCM medium (ScienCell cat# 5301) supplemented with 2% fetal bovine serum (FBS, ScienCell cat# 0010), 1% penicillin / streptomycin (ScienCell cat# 0503) and stellate cell growth supplement (SteCGS; ScienCell cat# 5352). Cell-culture flasks were coated with Poly-L Lysine (Sigma cat# P4707) for a better adherence.

### Preparation of compositions: 2 components combination matrix (FXR agonist/Elafibranor)

The FXR agonist INT-767 (CAS#1000403-03-1, Cat#HY-12434, batch#19249) was obtained commercially from Haoyuan Chemexpress. For these experiments, a checkerboard matrix was generated. INT-767 and Elafibranor were dissolved in dimethyl sulfoxide (DMSO, Fluka cat# 41640) and serially diluted in a 5-points series in a row (Elafibranor) and a 11-points series in a column (INT-767) of a 384-well plate. Subsequently, the 5X11 combination matrix was generated by 1: 1 mixing of all single agent concentrations. The test concentrations for each compound were chosen based on literature (Rizzo et al.; McMahan et al. ).

### Activation of hHSC with TGF-β1 and compound treatment

The hHSC were plated at a density of 6.5 × 10³cells/well into 384-well plates. The next day, cell-culture medium was removed, and cells were washed with PBS (Invitrogen cat# 14190). hHSC were deprived for 24 hours in serum-free and SteCGS-free medium. For the treatments with INT-767 and Elafibranor and their pairwise combinations, the serum-deprived hHSC were preincubated for 1 hour with the compounds followed by addition of the profibrogenic stimuli TGF-β1 (PeproTech cat# 100-21, 1 ng/mL) in serum-free and SteCGS-free medium for an additional 48 hour period.

### α-SMA ELISA

The level of α-SMA was measured using a Sandwich ELISA. Briefly, the wells of an ELISA plate were first coated with the capture antibody (mouse monoclonal anti-ACTA2, Abnova) at 4°C overnight. After 3 washes in PBS + 0,2% Tween 20, a blocking solution consisting of PBS +0.2% BSA was added for one hour followed by another washing cycle. The cell lysates were transferred into the wells for binding to the capture antibody for a period of 2h at room temperature. After the washing procedure, the detection antibody (biotinylated mouse monoclonal anti-ACTA2, Abnova) was added for 2 hours at room temperature followed by 3 washes. For the detection, an HRP-conjugated Streptavidin (R&D Systems cat# DY998) was first applied for 30 min at room temperature. After washing, the HRP substrate TMB (BD#555214) was added and incubated for 7min at room temperature in the dark. Upon oxidation, TMB forms a water-soluble blue reaction product that becomes yellow with addition of sulfuric acid (solution stop), enabling accurate measurement of the intensity at 450nm using a spectrophotometer. The developed color is directly proportional to the amount of α-SMA present in the lysate.

### Determination of synergism by Excess Over Bliss (EOB) method

The values obtained in the αSMA ELISA assays were first transformed into percentage inhibitions over TGF-β1 control. Then, using these percentage inhibitions, EOB (Excess Over Bliss) was determined to define the synergistic effects of drug combinations. Expected Bliss additivism score (E) was firstly determined by the equation:
E = (A + B) - (A × B) where A and B are the percentage inhibition of Elafibranor (A) and INT-767 (B) at a given dose. The difference between the Bliss expectation and the observed inhibition of the combined INT-767/Elafibranor at the same dose is the 'Excess over Bliss' score.
- Excess over Bliss score = 0 indicates that the combination treatment is additive (as expected for independent pathway effects);
- Excess over Bliss score >0 indicates activity greater than additive (synergy);
- Excess over Bliss score <0 indicates the combination is less than additive (antagonism).

For the combinations INT-767/Elafibranor, an additional total Bliss score was calculated by summation of all EOB.

To validate the synergism, the experimental values corresponding to top EOB score for FXR agonists/Elafibranor combination were plotted in a bar graph.

The significance of the observed differences between INT-767/Elafibranor over the highest single agent was determined by a student's t-test. * : p<0.05; **: p<0.01; ***: p<0.001.

### Results and discussion

### Evaluation of Elafibranor, OCA and the combination Elafibranor + OCA in a chronic CDAA + 1% cholesterol model (12 weeks)

The results are reported in the following table and in figures 1-3.

| | GFT505 | OCA | GFT505 + OCA |
|---|---|---|---|
| | 3mg/kg/d | 10mg/kg/d | |
| Fibrosis surface | 34% ± 17% *** | 74% ± 45% | **19% ± 4% #** |
| Hepatic collagen content | 45% ± 12% *** | 67% ± 23% ** | **34% ± 5% #** |
| α-SMA mRNA level | 66% ± 27% | 109% ± 68% | **39% ± 18% #** |
| TIMP1 mRNA level | 78% ± 23% | 110% ± 43% | **46% ± 13% ##** |
| TGFβ1 mRNA level | 94% ± 20% | 110% ± 23% | **67% ± 16% ##** |
| CCRS mRNA level⁺ | 103% ± 51% | 81% ± 28% | **56% ± 17% #** |

| | | | |
|---|---|---|---|
| *Percentage over the untreated CDAA* + *1% cholesterol rats* ^{∗∗} *p*<*0.01,* ^{∗∗∗} *p*<*0.001 vs CDAA* + *1% cholesterol group (ANOVA* + *Bonferroni)* *# p<0.05,* ## *p<0.01 vs the best single agent (Student t-test)* *(*+ *marker of inflammation)* | | | |

Western life style is invariably linked with high incidence rate of non-alcoholic steatohepatitis (NASH), a chronic liver disease that often progresses to liver fibrosis and cirrhosis and may ultimately lead to hepatocellular carcinoma. Currently, there is no approved therapy for NASH. Drug combinations directed simultaneously at multiple therapeutic targets have the potential to dramatically improve the drug response and to benefit the widest patient population. Drug combinations were previously tested in other systemic diseases, such as hypertension, dyslipidemia or type 2 diabetes and showed better control of the underlying diseases and decreased the morbidity and the mortality. In recent phase 2B studies, both Elafibranor (PPARα/δ agonist) and OCA (FXR agonist) have shown efficacy on NASH and fibrosis endpoints. We wanted to compare their action on relevant NASH pathology outcomes, and to look for therapeutic benefits of the combination.

To achieve this aim, fibrosing NASH was induced by feeding Wistar rats with a choline-deficient L-amino-acid-defined-diet that was supplemented with cholesterol (CDAA/chol diet). Animals in the intervention groups, received either Elafibranor or OCA or both compounds for the entire study period. NASH and fibrosis development were evaluated by histology. Additional biochemical and molecular analyses were also performed on different relevant biomarkers.

Wistar rats fed with the CDAA/chol diet developed NASH-related histology and fibrosis with high penetration of severe disease. Advanced steatosis, lobular inflammation and ballooning were present in all animals and NAS score [min 0 - max 8] varied between 6 and 8. Hepatic histology (picrosirius positive area) and biochemistry (hepatic collagen concentration) showed on average a fourfold increase in hepatic fibrosis content and fibrosis score was either 3 or 4 for all the animals on the CDAA/chol diet that received no drug treatment (Figures 1-2). The expression of genes related to inflammation, tissue remodeling and fibrosis was increased and consistent with gene signatures that were previously reported in NASH patients with severe disease (Figure 3).

Elafibranor administration alone attenuated fibrosis development, while OCA administration alone does not attenuate fibrosis in a significant manner (Figures 1-2). Combination treatments were more potent in reducing fibrosis, allowing reduction of doses, with similar efficacy on fibrosis achieved with both GFT505 1 and 3 mg/kg combined with OCA 10 mg/kg (Figures 1-2). The administration of either drug candidate alone only partially attenuated the increase of tissue remodeling, whereas the combination of both compounds was more efficient as compared to any single agent (Figure 3).

Therefore, it is herein shown that the synergistic action of Elafibranor and OCA on liver fibrosis in the CDAA/chol diet-induced NASH model produced a comparable therapeutic benefit at significantly lower doses of both drug candidates, as compared to any single agent. From this study, it is credibly expected that doses of both drug candidates can be lowered by a factor of at least 1.5, 2, 2.5 or even at least 3 to obtain results similar to the initial dose of each compound used individually. In addition, Elafibranor showed a clear protective effect on liver damage. The effects of OCA on ballooning and lobular inflammation were rather modest in this model. From this study, it can be concluded that Elafibranor/OCA combination would benefit a wider patient population and the associated therapeutic dose reduction would decrease the incidence of adverse drug effects.

### Evaluation of the combination of Elafibranor and another FXR agonist in a vitro model of hepatic fibrogenesis

The results are reported in figures 4-5.

The abnormal persistence of differentiated myofibroblasts is a characteristic of many fibrotic diseases. Following liver injury, quiescent HSCs undergo a process of activation that is characterized by a differentiation into (α-SMA)-positive myofibroblasts.

The PPAR agonist Elafibranor reveals an antifibrotic activity in hHSC activated with the profibrogenic cytokine TGFβ1. The α-SMA marker was reduced by up to 68% with the highest dose of Elafibranor tested (5µM) (Fig.4A). INT-767 alone only barely inhibited the production of α-SMA by 13% at the highest dose tested (30µM) (Fig.4B).. In order to evaluate if a combination of Elafibranor with INT-767 could reduce fibrosis in a synergistic manner, combination matrix experiments were performed in TGFβ-induced HSCs. Briefly, INT-767 and Elafibranor solutions were serially diluted in a checkerboard format generating a 55 combination matrix covering a large panel of INT-767/Elafibranor ratios. Synergy was first determined by calculating Excess Over Bliss scores. These experiments revealed that Elafibranor could synergize with INT-767 to reduce α-SMA production in activated HSCs (Fig. 5). One of the best example of synergy is shown in Fig.5C with 2.5µM of Elafibranor and 1.9µM of INT-767. Although 1.9µM of INT-767 alone does not show any antifibrotic activity, its addition to 2.5µM of Elafibranor increased synergistically the activity of Elafibranor and reached up to 69% of inhibition (compared to 26% with 2.5µM of Elafibranor alone). In conclusion, the synergistic action of Elafibranor and different classes of FXR agonists on fibrogenesis shows the potential benefit of such combinations in multiple types of fibrotic diseases.

### REFERENCES

Angulo P et al., 2002. Best Pract Res Clin Gastroenterol ; 16: 797-810.
Brunt EM et al, 1999, Am J Gastroenterol ;94(9):2467-74
Dowman J.K et al., 2010, Q J Med; 103: 71-83
Gressner A et al., 2009, World J Gastroenterol; 15: 2433-2440.
Kleiner DE et al, 2005, Hepatology;41(6):1313-21
Marchesini G et al.2003. Hepatology; 37:917-923.
McMahan et al. , 2013, The Journal of Biological Chemistry 288(17): 11761-11770
Nelson A et al., 2009. J Clin Gastroenterol; 43: 990-994
Neuschwander-Tetri et al., 2003. Hepatology; 38: 1008-1017.
Yeh M et al., 2007. Am J Clin Pathol; 128:837-847.
Rizzo et al. 2010, Molecular Pharmacology 78:617-630
Shiri-Sverdlov R et al., 2006. J Hepatol; 44: 732-41.
Vuppalanchi Rand Chalasani N, 2009. Hepatology; 49: 306-317.

## Claims

1. A combination product comprising:
(i) Elafibranor (ELA) or a pharmaceutically acceptable salt thereof; and
(ii) a FXR agonist, wherein the FXR agonist is Obeticholic acid (OCA) or a pharmaceutically acceptable salt thereof, or INT-767 or a pharmaceutically acceptable salt thereof..

2. The combination product according to claim 1, wherein the FXR agonist is Obeticholic acid (OCA) or a pharmaceutically acceptable salt thereof.

3. The combination product according to claim 1, wherein the FXR agonist is INT-767 or a pharmaceutically acceptable salt thereof.

4. The combination product according to any one of claims 1 to 3, wherein the combination product is a composition comprising (i) and (ii) and a pharmaceutically acceptable carrier.

5. The combination product according to any one of claims 1 to 3, wherein the combination product is a kit of parts comprising (i) and (ii).

6. The combination product according to any one of claims 1 to 5, wherein (i) and (ii) are at a synergistic effective amount for the treatment of a disease.

7. The combination product according to any one of claims 1 to 6, for use in a method for the treatment of an immune, inflammatory, metabolic, fibrotic or cholestatic disease.

8. The combination product for use according to claim 7, wherein the disease is NAFLD, NASH, liver fibrosis, liver cirrhosis, PBC or PSC.

9. The combination product for use according to claim 7 or 8, wherein:
- ELA is administered at a dose comprised between 5 and 80 mg/day and OCA is administered at a dose comprised between 7 and 17 mg/day;
- ELA is administered at a dose comprised between 40 and 60 mg/day and OCA is administered at a dose comprised between 5 and 12.5 mg/day;
- ELA is administered at a dose comprised between 32 and 48 mg/day and OCA is administered at a dose comprised between 4 and 10 mg/day;
- ELA is administered at a dose comprised between 27 and 40 mg/day and OCA is administered at a dose comprised between 3 and 8 mg/day;
- ELA is administered at a dose comprised between 16 and 24 mg/day and OCA is administered at a dose comprised between 2 and 5 mg/day; or
- ELA is administered at a dose comprised between 8 and 12 mg/day and OCA is administered at a dose comprised between 1 and 2.5 mg/day.

## Patentansprüche

1. Kombinationsprodukt enthaltend:
(i) Elafibranor (ELA) oder ein pharmazeutisch verträgliches Salz davon; und
(ii) aein FXR-Agonist, wobei der FXR-Agonist Obeticholsäure (OCA) oder ein pharmazeutisch verträgliches Salz davon oder INT-767 oder ein pharmazeutisch verträgliches Salz davon ist.

2. Kombinationsprodukt nach Anspruch 1, wobei der FXR-Agonist Obeticholsäure (OCA) oder ein pharmazeutisch verträgliches Salz davon ist.

3. Kombinationsprodukt nach Anspruch 1, wobei der FXR-Agonist INT-767 oder ein pharmazeutisch verträgliches Salz davon ist.

4. Kombinationsprodukt nach einem der Ansprüche 1 bis 3, wobei das Kombinationsprodukt eine Zusammensetzung ist, die (i) und (ii) und einen pharmazeutisch verträglichen Träger enthält.

5. Kombinationsprodukt nach einem der Ansprüche 1 bis 3, wobei das Kombinationsprodukt ein Kit aus Teilen ist, das (i) und (ii) enthält.

6. Kombinationsprodukt nach einem der Ansprüche 1 bis 5, wobei (i) und (ii) in einer synergistisch wirksamen Menge zur Behandlung einer Krankheit vorliegen.

7. Kombinationsprodukt nach einem der Ansprüche 1 bis 6 zur Verwendung in einem Verfahren zur Behandlung einer Immun-, Entzündungs-, Stoffwechsel-, fibrotischen oder cholestatischen Erkrankung.

8. Kombinationsprodukt zur Verwendung nach Anspruch 7, wobei es sich bei der Erkrankung um NAFLD, NASH, Leberfibrose, Leberzirrhose, PBC oder PSC handelt.

9. Kombinationsprodukt zur Verwendung nach Anspruch 7 oder 8, wobei:
- ELA wird in einer Dosis zwischen 5 und 80 mg/Tag verabreicht und OCA wird in einer Dosis zwischen 7 und 17 mg/Tag verabreicht;
- ELA wird in einer Dosis zwischen 40 und 60 mg/Tag verabreicht und OCA wird in einer Dosis zwischen 5 und 12,5 mg/Tag verabreicht;
- ELA wird in einer Dosis zwischen 32 und 48 mg/Tag verabreicht und OCA wird in einer Dosis zwischen 4 und 10 mg/Tag verabreicht;
- ELA wird in einer Dosis zwischen 27 und 40 mg/Tag verabreicht und OCA wird in einer Dosis zwischen 3 und 8 mg/Tag verabreicht;
- ELA wird in einer Dosis zwischen 16 und 24 mg/Tag verabreicht und OCA wird in einer Dosis zwischen 2 und 5 mg/Tag verabreicht; oder
- ELA wird in einer Dosis zwischen 8 und 12 mg/Tag verabreicht und OCA wird in einer Dosis zwischen 1 und 2,5 mg/Tag verabreicht.

## Revendications

1. Produit de combinaison comprenant :
(i) de l'élafibranor (ELA) ou un sel pharmaceutiquement acceptable de celui-ci ; et
(ii) un agoniste de FXR, lequel agoniste de FXR est l'acide obéticholique (OCA) ou un sel pharmaceutiquement acceptable de celui-ci, ou l'INT-767 ou un sel pharmaceutiquement acceptable de celui-ci.

2. Produit de combinaison selon la revendication 1, dans lequel l'agoniste de FXR est l'acide obéticholique (OCA) ou un sel pharmaceutiquement acceptable de celui-ci.

3. Produit de combinaison selon la revendication 1, dans lequel l'agoniste de FXR est l'INT-767 ou un sel pharmaceutiquement acceptable de celui-ci.

4. Produit de combinaison selon l'une quelconque des revendications 1 à 3, lequel produit de combinaison est une composition comprenant (i) et (ii) et un véhicule pharmaceutiquement acceptable.

5. Produit de combinaison selon l'une quelconque des revendications 1 à 3, lequel produit de combinaison est un kit comprenant (i) et (ii).

6. Produit de combinaison selon l'une quelconque des revendications 1 à 5, dans lequel (i) et (ii) sont en une quantité efficace synergique pour le traitement d'une maladie.

7. Produit de combinaison selon l'une quelconque des revendications 1 à 6, pour une utilisation dans une méthode pour le traitement d'une maladie immune, inflammatoire, métabolique, fibrotique ou cholestatique.

8. Produit de combinaison pour une utilisation selon la revendication 7, dans lequel la maladie est une NAFLD, une NASH, une fibrose hépatique, une cirrhose hépatique, une PBC ou une PSC.

9. Produit de combinaison pour une utilisation selon la revendication 7 ou 8, dans lequel :
- ELA est administré à une dose comprise entre 5 et 80 mg/jour et OCA est administré à une dose comprise entre 7 et 17 mg/jour ;
- ELA est administré à une dose comprise entre 40 et 60 mg/jour et OCA est administré à une dose comprise entre 5 et 12,5 mg/jour ;
- ELA est administré à une dose comprise entre 32 et 48 mg/jour et OCA est administré à une dose comprise entre 4 et 10 mg/jour ;
- ELA est administré à une dose comprise entre 27 et 40 mg/jour et OCA est administré à une dose comprise entre 3 et 8 mg/jour ;
- ELA est administré à une dose comprise entre 16 et 24 mg/jour et OCA est administré à une dose comprise entre 2 et 5 mg/jour ; ou
- ELA est administré à une dose comprise entre 8 et 12 mg/jour et OCA est administré à une dose comprise entre 1 et 2,5 mg/jour.
